# EUROPEAN PATENT APPLICATION

(11) **EP 4 725 319 A1**
(43) Date of publication of application: **15.04.2026**
(21) Application number: 24819426.8
(22) Date of filing: 07.06.2024
(51) Int. Cl.: A23L 29/00, A23L 5/41, A61K 9/10, A61K 9/19, A61K 31/661, A61K 47/02, A61P 3/02

(54) **COMPOSITION COMPRISING SELF-ASSEMBLED STRUCTURE, AND FREEZE-DRIED PRODUCT THEREOF**

(30) Priority: 08.06.2023 JP 2023095106
(71) Applicant: Resonac Corporation, Tokyo 105-7325 (JP)
(72) Inventor: OHTAKE, Noriko, Tokyo 105-7325 (JP); YAMASHITA, Yuji, Yokohama-shi, Kanagawa 221-8686 (JP)
(74) Representative: Strehl & Partner mbB
(86) International application number: PCT/JP2024/020938
(87) International publication number: WO 2024/253199

(57) **Abstract**

A composition containing a self-assembled structure of a tocopheryl phosphate, a di- or higher valent metal salt, and water. In addition, a freeze-dried product containing a self-assembled structure of a tocopheryl phosphate and a di- or higher valent metal salt, in which the mole ratio of the di- or higher valent metal salt to the tocopheryl phosphate is 0.05 to 12.

## Description

### TECHNICAL FIELD

The present invention relates to a composition containing a self-assembled structure and a freeze-dried product thereof.

Priority is claimed on Japanese Patent Application No. 2023-095106, filed June 8, 2023, the content of which is incorporated herein by reference.

### BACKGROUND ART

A tocopheryl phosphate is an amphiphilic compound containing a phosphate group introduced into oil-soluble vitamin E. A mixed composition of sodium tocopheryl phosphate, which is a sodium salt of a tocopheryl phosphate, (hereinafter also referred to as "TPNa") and a phospholipid has been reported to form a nanostructure "tocosome" and is being studied as a delivery carrier of nutritional supplements and medication (Non-Patent Document 1)

In recent studies, it has been clarified that TPNa self-assembles in water even on its own to create a novel mitochondrion-like structure (Non-Patent Document 2), and there is an expectation of the industrial use of this structure.

### Citation List

### Non-Patent Document

Non-Patent Document 1: M.R. Mozafari, et al., Tocosome: Novel drug delivery system containing phospholipids and tocopheryl phosphates. International Journal of Pharmaceutics. Volume 528, Issues 1-2, 7 August 2017, Pages 381 and 382
Non-Patent Document 2: Yuji Yamashita et al. "Unique Self-Assembly Behavior of Vitamin E Derivative Aqueous Solution" Proceedings of The 73rd Divisional Meeting of Division of Colloid and Surface Chemistry

### SUMMARY OF INVENTION

### Technical Problem

A tocopheryl phosphate forms a self-assembled structure in water, but the self-assembled structure collapses when the tocopheryl phosphate coexists with a polar organic compound, such as an alcohol. In order to put the self-assembled structure of the tocopheryl phosphate into practical use, it is desired that the self-assembled structure can be stably maintained even under the coexistence of a polar organic compound.

Therefore, an object of the present invention is to provide a composition containing a self-assembled structure of a tocopheryl phosphate in which the self-assembled structure is stably maintained and a dried product of the composition. Solution to Problem

The present invention includes the following aspects.
[1] A composition containing a self-assembled structure of a tocopheryl phosphate, a di- or higher valent metal salt, and water.
[2] The composition according to [1], in which the di- or higher valent metal salt is at least one or more selected from the group consisting of a calcium salt, a magnesium salt, and a zinc salt.
[3] The composition according to [1] or [2], in which the concentration of the tocopheryl phosphate is 0.001 mM to 100 mM, and the mole ratio of the di- or higher valent metal salt to the tocopheryl phosphate is 0.05 to 12.
[4] A composition containing a self-assembled structure of a tocopheryl phosphate and a di- or higher valent metal salt, in which the mole ratio of the di- or higher valent metal salt to the tocopheryl phosphate is 0.05 to 12, and the water content is 1.0 mass% or less.
[5] A freeze-dried product of the composition according to any one of [1] to [3].

### Advantageous Effects of Invention

According to the present invention, a composition containing a self-assembled structure of a tocopheryl phosphate in which the self-assembled structure is stably maintained and a dried product of the composition are provided.

### BRIEF DESCRIPTION OF DRAWINGS

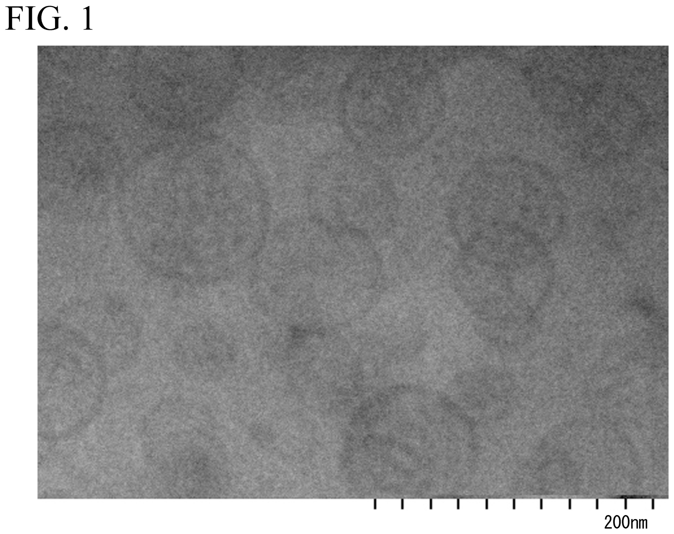

[FIG. 1] A photograph of a self-assembled structure of Example 2 observed with a transmission electron microscope (Cryo-TEM) at a magnification of 20000 times, the scale bar being 200 nm.

### DESCRIPTION OF EMBODIMENTS

Hereinafter, an embodiment of the present invention will be described in detail. However, the present invention is not limited to the embodiment to be described below. In the present specification, in the case of using "to" regarding a numerical range, numerical values at both ends are the upper limit value and the lower limit value, respectively and are included in the numerical range.

### Composition

In one embodiment, the present disclosure provides a composition. The composition of the present embodiment contains, in addition to a self-assembled structure of a tocopheryl phosphate, a di- or higher valent metal salt and water.

### Self-assembled structure of tocopheryl phosphate

A tocopheryl phosphate is a compound containing a phosphate group introduced into a hydroxy group of tocopherol. Examples of the tocopheryl phosphate include compounds represented by the following general formula (1).

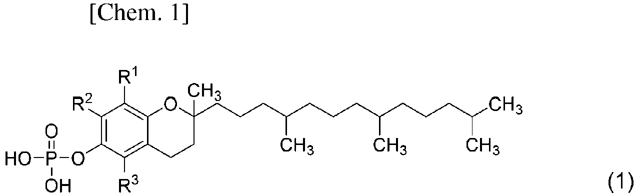

[In the formula, R¹, R², and R³ each independently represent a hydrogen atom or a methyl group.]

In the tocopheryl phosphate, depending on the kinds of R¹, R², and R³ in the general formula (1), α-tocopheryl phosphate (R¹, R², and R³ = CH₃), β-tocopheryl phosphate (R¹ and R³ = CH₃, R² = H), γ-tocopheryl phosphate (R¹ and R² = CH₃, R³ = H), δ-tocopheryl phosphate (R¹ = CH₃, R² and R³ = H), ζ₂-tocopheryl phosphate (R² and R³ = CH₃, R¹ = H), η-tocopheryl phosphate (R² = CH₃, R¹ and R³ = H), and the like are present.

The tocopheryl phosphate may be any of these tocopheryl phosphates. Among these, α-tocopheryl phosphate or γ-tocopheryl phosphate is preferable, and α-tocopheryl phosphate is more preferable.

The compound represented by the following general formula (1) has an asymmetric carbon atom at the second position of a chroman ring and is thus present as stereoisomers of a d form and an l form and a dl form. The tocopheryl phosphate may be any of these stereoisomers, but the dl form is preferable.

Among these, as the tocopheryl phosphate, dl-α-tocopheryl phosphate and dl-γ-tocopheryl phosphate are preferable, and dl- α-tocopheryl phosphate is more preferable.

The tocopheryl phosphate that forms the self-assembled structure preferably dissolves in water or an aqueous solution as an alkali metal salt. Examples of the alkali metal salt of the tocopheryl phosphate include a sodium salt of the tocopheryl phosphate and a potassium salt of the tocopheryl phosphate, and the sodium salt is more preferable. The sodium salt of the tocopheryl phosphate has advantages of high solubility in water and a powdery nature, which makes the sodium salt easy to handle.

The alkali metal salt of the tocopheryl phosphate may be used singly or two or more thereof may be used in combination.

As the alkali metal salt of the tocopheryl phosphate, a commercially available product may be used or the alkali metal salt may be produced by a well-known method. A sodium salt of the dl-α-tocopheryl phosphate has been put on the market by Resonac Corporation under the product name of TPNa (registered trademark) (display name: Na tocopheryl phosphate).

In the case of producing the alkali metal salt of the tocopheryl phosphate, examples of a well-known production method include the methods described in Japanese Unexamined Patent Application, First Publication No. H59-44375, WO97/14705, and the like. Specifically, the tocopheryl phosphate can be obtained by causing a phosphorylating agent, such as phosphorus oxychloride, to act on tocopherol dissolved in a solvent and appropriately purifying a reaction product after the end of a reaction. Furthermore, the obtained tocopheryl phosphate is neutralized with an alkali metal hydroxide, such as sodium hydroxide, whereby an alkali metal salt of the tocopheryl phosphate can be obtained.

"Self-assembled structure of tocopheryl phosphate" refers to a structure having a bag-like structure of a bilayer membrane that is formed by the association of a plurality of tocopheryl phosphate molecules in water. Such a self-assembled structure is also generally referred to as a vesicle.

In "self-assembled structure of tocopheryl phosphate," the majority of the tocopheryl phosphate is present as tocopheryl phosphate ions. When the alkali metal salt of the tocopheryl phosphate is dissolved in water, normally, the alkali metal salt of the tocopheryl phosphate is dissociated into tocopheryl phosphate ions and alkali metal ions, but there is a case where an extremely small amount of a part of the alkali metal salt of the tocopheryl phosphate becomes a nonionized tocopheryl phosphate. The self-assembled structure is formed of such tocopheryl phosphate ions and nonionized tocopheryl phosphate. In the present specification, the nonionized tocopheryl phosphate and the tocopheryl phosphate ions will be collectively referred to as "tocopheryl phosphate" in some cases.

When the pH is 4 or higher, in the tocopheryl phosphate present in the composition, the the ratio of the nonionized tocopheryl phosphate is low, and consequently it becomes easy to form the self-assembled structure. Therefore, the composition of the present embodiment preferably has a pH of 4 or higher. The composition more preferably has a pH of 5 or higher and still more preferably 6 or higher.

In a case where the pH is 12 or lower, the hydrolysis speed of a phosphate moiety in the structure of the tocopheryl phosphate becomes slow, and the temporal stability of the composition thus improves. Therefore, the composition of the present embodiment preferably has a pH of 12 or lower. The composition more preferably has a pH of 11 or lower and still more preferably 10 or lower.

The upper limit values and lower limit values can be arbitrarily combined. The pH of the composition is preferably 4 to 12, more preferably 5 to 11, and still more preferably 6 to 10.

The concentration of the tocopheryl phosphate in the composition of the present embodiment is preferably 0.001 mM or higher, more preferably 0.01 mM or higher, and still more preferably 0.1 mM. The concentration of the tocopheryl phosphate is preferably 100 mM or lower, more preferably 40 mM or lower, and still more preferably 10 mM or lower. The upper limit values and lower limit values can be arbitrarily combined. The concentration of the tocopheryl phosphate in the composition of the present embodiment is preferably 0.001 to 100 mM, more preferably 0.01 to 40 mM, and still more preferably 0.1 to 10 mM. When the concentration of the tocopheryl phosphate in the composition is 0.001 mM or higher, the self-assembled structure of the tocopheryl phosphate is likely to be formed. When the concentration of the tocopheryl phosphate in the composition is 100 mM or lower, liquid crystal phase transition rarely occurs. When the concentration of the tocopheryl phosphate in the composition of the present embodiment is within a range of 0.001 to 100 mM, the self-assembled structure of the tocopheryl phosphate is likely to be formed. "mM" means mmol/L.

### Di- or higher valent metal salt

As the di- or higher valent metal salt, a di- or trivalent metal salt is preferable, and a divalent metal salt is more preferable. Examples of the divalent metal salt include calcium salts, magnesium salts, zinc salts, aluminum salts, iron salts, copper salts, and the like. Among these, the di- or higher valent metal salt is preferably at least one selected from the group consisting of a calcium salt, a magnesium salt, and a zinc salt for high biocompatibility. More specifically, the di- or higher valent metal salt is preferably at least one selected from the group consisting of calcium chloride, magnesium chloride, and zinc chloride.

Regarding the content of the di- or higher valent metal salt in the composition of the present embodiment, the mole ratio of the di- or higher valent metal salt to 1 mol of the tocopheryl phosphate is preferably 0.05 or more. The mole ratio of the di- or higher valent metal salt to 1 mol of the tocopheryl phosphate is preferably 12 or less, more preferably 1 or less, and still more preferably 0.2 or less. The upper limit values and lower limit values can be arbitrarily combined. The mole ratio of the di- or higher valent metal salt to 1 mol of the tocopheryl phosphate is preferably 0.05 to 12, more preferably 0.05 to 1, and still more preferably 0.05 to 0.2. When the mole ratio of the di- or higher valent metal salt to the tocopheryl phosphate is 0.05 or more, the effect of the di- or higher valent metal salt stabilizing the self-assembled structure becomes higher. When the mole ratio of the di- or higher valent metal salt to the tocopheryl phosphate is 12 or less, the amount of the di- or higher valent metal salt is less likely to be excessive. In a case where the mole ratio of the di- or higher valent metal salt to the tocopheryl phosphate is larger than 1, there is a case where the tocopheryl phosphate is precipitated due to a salting-out effect depending on the concentration of the tocopheryl phosphate and/or the kind of the metal salt. Therefore, in a case where the mole ratio of the di- or higher valent metal salt to the tocopheryl phosphate is set to larger than 1 (preferably 12 or less), it is preferable to appropriately select the concentration of the tocopheryl phosphate and the kind of the di- or higher valent metal salt.

### Water

As the water, for example, pure water, ultrapure water, ion-exchanged water, purified water, sterile purified water, water for injection, and the like can be used depending on the use. As the water, cosmetic-grade or pharmaceutical-grade water is preferably used.

The content of the water in the composition of the present embodiment can be set to the remaining amount of the total amount of the alkali metal salt of the tocopheryl phosphate, the di- or higher valent metal salt, and an arbitrary component. The content of the water in the composition of the present embodiment is normally 70 mass% or more and less than 100 mass%, preferably 75 mass% or more and less than 100 mass%, more preferably 80 mass% or more and less than 100 mass%, and still more preferably 85 mass% or more and less than 100 mass% relative to the total mass of the composition.

In a case where a compound that forms a self-assembled structure on its own other than the tocopheryl phosphate is contained in the composition in a large quantity, the composition is no longer the intended composition of the present invention, that is, a composition in which a self-assembled structure also having the characteristics of a tocopheryl phosphate is stably maintained. Therefore, the content of the compound that forms a self-assembled structure on its own other than the tocopheryl phosphate in the composition of the present embodiment is preferably 50 mass% or less, more preferably 30 mass% or less, and still more preferably 10 mass% or less when the total mass of the compounds that form the self-assembled structure is set to 100 mass%. The content of the compound that forms a self-assembled structure on its own other than the tocopheryl phosphate in the composition of the present embodiment is preferably 0 mass% and may be 0 to 50 mass%, may be 0 to 30 mass%, or may be 0 to 10 mass% relative to the total mass of the compounds that form the self-assembled structure (100 mass%).

### Arbitrary component

The composition of the present embodiment may contain an arbitrary component. The arbitrary component is not particularly limited as long as the self-assembled structure is maintained. Examples of the arbitrary component include polar organic compounds, drugs or additives that are commonly used in pharmaceuticals or cosmetics, and the like.

Examples of the polar organic compounds include alcohols. Specific examples of the alcohols include monovalent alcohols, such as methanol, ethanol, 1-propanol, isopropanol, and 1-butanol; divalent alcohols such as 1,3-butylene glycol and ethylene glycol; and the like.

Examples of the drugs or additives include nutrients, preservatives, antibacterial agents, skin whitening agents, vitamins and derivatives thereof, antiphlogistic, anti-inflammatory agents, blood circulation promoters, hormones, anti-wrinkle agents, anti-aging agents, tightening agents, cooling agents, warming agents, wound healing promoters, irritation relief agents, analgesics, cell activators, plant/animal/microbial extracts, antipruritic agents, keratin peeling/dissolving agents, astringents, enzymes, nucleic acids, fragrances, pigments, colorants, anti-inflammatory analgesics, antifungal agents, antihistamines, antibiotics, antibacterial substances, herbal medicines, antipruritic agents, keratin softening/peeling agents, preservatives and disinfectants, antioxidants, pH adjusters, other additives, and the like.

The shape of the self-assembled structure of the tocopheryl phosphate can be confirmed by observing the self-assembled structure with a transmission electron microscope or the like. Therefore, whether or not the tocopheryl phosphate has formed a self-assembled structure under a condition that the arbitrary component is contained in the composition can be confirmed by observing the composition with a transmission electron microscope or the like.

The average particle diameter of the self-assembled structure can be measured by arbitrary measuring methods. For the measurement of the average particle diameter in the self-assembled structure, for example, a dynamic light scattering measurement method can be simply used. The use of the dynamic light scattering measurement method makes it possible to find out a change in the average particle diameter, which may be caused by a condision such as a storage condision of the composition, by measuring the average particle diameters of the self-assembled structure before and after a condition such as a storage condition is changed. When the change in the average particle diameter observed at this time is not large, it is possible to find out that the self-assembled structure is stable under the storage condition. For example, when it is necessary to find out an influence of the addition of the arbitrary component, a change in the average particle diameter in the self-assembled structure before and after the addition of the arbitrary component needs to be observed. Normally, in a case where the self-assembled structure is not stable, the average particle diameter significantly decreases due to the addition of the polar organic compound or the like, and it is thus possible to find out that the self-assembled structure has been dissociated.

Therefore, it is possible to select, as the arbitrary component, a component that causes only a small change in the average particle diameter in the self-assembled structure of the tocopheryl phosphate before and after the addition of the arbitrary component to the composition. In a case where the above-described arbitrary component and the polar organic compound have been added to the composition as the arbitrary components, a component that causes only a small change in the average particle diameter in the self-assembled structure of the tocopheryl phosphate is more preferable.

### Method for producing composition

The composition of the present embodiment can be produced by dissolving the alkali metal salt of the tocopheryl phosphate and the di- or higher valent metal salt in water. In a case where the composition of the present embodiment contains the arbitrary component, the arbitrary component also needs to be dissolved in water. The alkali metal salt of the tocopheryl phosphate and the di- or higher valent metal salt may be dissolved in water at the same time or may be dissolved in water in order. Alternatively, an aqueous solution of the alkali metal salt of the tocopheryl phosphate and an aqueous solution of the di- or higher valent metal salt are each prepared, and these aqueous solutions are mixed together. Between these methods, the method in which the individual aqueous solutions are prepared and both are mixed together is preferable since the alkali metal salt of the tocopheryl phosphate and the di- or higher valent metal salt can be uniformly dissolved. In a method in which the alkali metal salt of the tocopheryl phosphate is added to an aqueous solution containing the di- or higher valent metal salt dissolved therein, there is a case where an undissolved residue of the alkali metal salt of the tocopheryl phosphate is generated. In a method in which the di- or higher valent metal salt is added to and dissolved in an aqueous solution of the alkali metal salt of the tocopheryl phosphate, there is a case where the di- or higher valent metal salt does not uniformly dissolve, becomes cloudy, and is precipitated.

Since the Krafft point (Kp) of a TPNa aqueous solution is 16.2°C, in the case of using TPNa as the alkali metal salt of the tocopheryl phosphate, the composition of the present embodiment is preferably produced at a temperature of 17°C or higher and more preferably produced at a temperature of 20°C to 80°C. In a case where the alkali metal salt of the tocopheryl phosphate is not a sodium salt as well, the composition of the present embodiment is preferably produced at a temperature that is equal to or higher than the Kraft point of each alkali metal salt.

It is also possible to incorporate an arbitrary compound (target compound) in the self-assembled structure of the tocopheryl phosphate. For example, it is possible to produce the self-assembled structure of the tocopheryl phosphate in which the target compound has been incorporated by adding and dissolving the alkali metal salt of the tocopheryl phosphate to and in an aqueous solution containing the target compound dissolved therein and mixing the aqueous solution with an aqueous solution of the di- or higher valent metal salt. After the production of the self-assembled structure, the self-assembled structure may be purified filtration, column chromatography, ultracentrifugation, or the like.

When a nutritional ingredient (for example, vitamins, amino acids, peptides, sugars, or the like), medication (an anticancer drug, an enzyme, an inhibitor, a hormone, a cytokine, or the like), or the like is used as the target compound, it is possible to use the self-assembled structure of the tocopheryl phosphate as a delivery carrier of nutritional supplements and medication. Into the self-assembled structure, an arbitrary component, such as an additive as described above, can be incorporated as appropriate.

The composition of the present embodiment normally has a form in which the self-assembled structure of the tocopheryl phosphate has been dispersed in an aqueous solution of the di- or higher valent metal salt. The composition of the present embodiment can also be said to be an aqueous solution containing the self-assembled structure of the tocopheryl phosphate dispersed therein or can also be said to be a dispersion of the self-assembled structure of the tocopheryl phosphate.

The composition of the present embodiment contains the di- or higher valent metal salt and is thus capable of stably maintaining the self-assembled structure of the tocopheryl phosphate. Conventional self-assembled structures of tocopheryl phosphates are not capable of maintaining the self-assembled structures in the presence of a polar organic compound, such as an alcohol (for example, ethanol, 1,3-butylene glycol, or the like). However, when the di- or higher valent metal salt is caused to coexist with the self-assembled structure of the tocopheryl phosphate, it is possible to stably maintain the self-assembled structure of the tocopheryl phosphate. The reason therefor is not certain but is considered that a di- or higher valent metal ion fulfills a function of coating the periphery of the self-assembled structure due to an interaction between the phosphate group of the tocohperyly phosphate that is positioned at the outer side of the self-assembled structure and the di- or higher valent metal ion.

The self-assembled structure of the tocopheryl phosphate that maintains the shape even in the presence of an alcohol or the like is not only available for the preparation of a self-assembled structure in which a useful chemical substance is retained but can also be used in more situations when used.

### Freeze-dried product of self-assembled structure

In one embodiment, the present disclosure provides a freeze-dried product of a self-assembled structure. The freeze-dried product of the present embodiment contains a self-assembled structure of a tocopheryl phosphate and a di- or higher valent metal salt. The mole ratio of the di- or higher valent metal salt to the tocopheryl phosphate is 0.05 to 12.

In addition, the freeze-dried product of the present embodiment may be a freeze-dried product of the composition of the present embodiment that is obtained by the freeze drying of the composition of the embodiment. Freeze drying is a method in which an object is frozen and then dried by sublimating water in a vacuum state. The freeze drying of the composition of the embodiment can be performed by a well-known method using a freeze dryer. The water content of the freeze-dried product after the freeze drying is preferably 1.0 mass% or less and more preferably 0.6 mass% or less. The water content of the freeze-dried product after the freeze drying can be measured by a Karl Fischer method or the like. The lower limit value of the water content of the freeze-dried product may be, for example, 0.001 mass% or more or 0.01 mass% or more. The range of the moisture content of the freeze-dried product is, for example, 0.001 to 1.0 mass% and preferably 0.001 to 0.6 mass%.

A powder containing the self-assembled structure of the tocopheryl phosphate can be obtained by the freeze drying of the composition of the embodiment. In the freeze-dried product, the self-assembled structure of the tocopheryl phosphate is maintained, and the freeze-dried product can also be used in the same uses as those of the composition. In addition, the freeze-dried product is a powder and is thus advantageous in terms of handling.

### Examples

Hereinafter, the present invention will be more specifically described with examples and comparative examples, but the present invention is not limited to the following examples.

### Example 1

0.32 g of TPNa (manufactured by Resonac Corporation) was put into a 100 mL volumetric flask and dissolved by adding ultrapure water (MilliQ) thereto (6 mM TPNa aqueous solution). 0.061 g of MgCl₂·6H₂O (manufactured by Junsei Chemical Co., Ltd.) was put into another 100 mL volumetric flask and dissolved by adding ultrapure water thereto (3 mM MgCl₂ aqueous solution). 12.5 g of the 6 mM TPNa aqueous solution, 2.5 g of the 3 mM MgCl₂ aqueous solution, both of which were prepared above, and 10 g of ultrapure water were put into a vial bottle and mixed together, thereby obtaining a mixed liquid containing 3 mM TPNa and 0.3 mM MgCl₂.

0.95 mL of the mixed liquid was weighed, and 0.05 mL of ethanol (manufactured by FUJIFILM Wako Pure Chemical Corporation) was added thereto (the content of ethanol reached approximately 5 mass%) and uniformly mixed at room temperature. After the mixed liquid was left to stand for one day, dynamic light scattering (DLS) measurement of the mixed liquid was performed (with Zetasizer, manufactured by Spectris Co., Ltd.) to confirm the formation of a self-assembled structure of TPNa. The mixed liquid was measured while being diluted, the particle size distribution was analyzed from autocorrelation functions, and the number average of particle diameters was calculated.

### Example 2

An experiment was performed by the same procedure as in Example 1 except that 0.044 g of CaCl₂·2H₂O (manufactured by Junsei Chemical Co., Ltd.) was used instead of MgCl₂·6H₂O.

### Comparative Example 1

An experiment was performed by the same procedure as in Example 1 except that ultrapure water was used instead of a 3 mM MgCl₂·6H₂O aqueous solution.

### Example 3

An experiment was performed by the same procedure as in Example 1 except that 1,3-butyrene glycol (1,3-BG, HAISUGARCANE BG, manufactured by Kokyu Alcohol Kogyo Co., Ltd.) was used instead of ethanol.

### Example 4

An experiment was performed by the same procedure as in Example 2 except that 1,3-BG was used instead of ethanol.

### Comparative Example 2

An experiment was performed by the same procedure as in Comparative Example 1 except that 1,3-BG was used instead of ethanol.

### Example 5

An experiment was performed by the same procedure as in Example 1 except that 1.25 g of the 6 mM TPNa aqueous solution and 0.25 g of the 3 mM MgCl₂ aqueous solution were added to 27 g of ultrapure water to obtain a mixed liquid containing 0.3 mM TPNa and 0.03 mM MgCl₂.

### Example 6

An experiment was performed by the same procedure as in Example 5 except that 0.044 g of CaCl₂·2H₂O (manufactured by Junsei Chemical Co., Ltd.) was used instead of MgCl₂·6H₂O.

The mixed liquids prepared in Examples 1 to 6 and Comparative Examples 1 and 2 were observed with a transmission electron microscope, the formation of a self-assembled structure was confirmed, and the evaluation results according to the following evaluation standards were shown in "Self-assembled structure" of Table 1. FIG. 1 shows a transmission electron microscope (Cryo-TEM) image (magnification: 20000 times) of the mixed liquid prepared in Example 2.

### Evaluation standards

A: A self-assembled structure was observed.
B: A self-assembled structure was not observed.

The results of calculating the number averages of particle diameters in the mixed liquids of Examples 1 to 6 and Comparative Examples 1 and 2 based on the results of the dynamic light scattering measurement are shown in "Particle diameter average (nm)" of Table 1.

**[Table 1]**

| | Example 1 | Example 2 | Comparative Example 1 | Example 3 | Example 4 | Comparative Example 2 | Example 5 | Example 6 |
|---|---|---|---|---|---|---|---|---|
| TPNa (mM) | 3 | 3 | 3 | 3 | 3 | 3 | 0.3 | 0.3 |
| MgCl₂ (mM) | 0.3 | - | - | 0.3 | | - | 0.03 | - |
| CaCl₂ (mM) | - | 0.3 | - | - | 0.3 | - | - | 0.03 |
| Mole ratio of metal salt/TPNa | 0.1 | 0.1 | - | 0.1 | 0.1 | - | 0.1 | 0.1 |
| EtOH (mass%) | 5 | 5 | 5 | - | - | - | 5 | 5 |
| 1,3-BG (mass%) | - | - | - | 5 | 5 | 5 | - | - |
| Self-assembled structure | A | A | B | A | A | B | A | A |
| Particle diameter average (nm) | 58.07 | 123.63 | 2.86 | 59.29 | 80.18 | 3.40 | 79.58 | 104.70 |

As a result of the dynamic light scattering measurement, in Comparative Examples 1 and 2, the particle diameters became small due to the addition of ethanol or 1,3-BG (= the self-assembled structures were dissociated). In contrast, in Examples 1 to 4, in the particle diameter did not change, and it was suggested that the self-assembled structures were stably maintained. From Examples 5 and 6, it was possible to confirm the formation of a self-assembled structure in spite of the fact that the concentrations of TPNa and the di- or higher valent metal salt were lower.

### Example 7

A 6 mM TPNa aqueous solution and a 3 mM MgCl₂ aqueous solution were prepared in the same manner as in Example 1. The 6 mM TPNa aqueous solution and the 3 mM MgCl₂ aqueous solution were put into a vial bottle and mixed together so that the final concentration of TPNa reached 3 mM and the final concentration of MgCl₂ reached 0.15 mM, 0.2 mM, 0.3 mM, 0.4 mM, 0.6 mM, 1.2 mM, 3 mM, 6 mM, or 12 mM. The appearances of mixed solutions were visually observed and determined based on the following evaluation standards, and the results thereof were shown in "Determination" of Table 2.

### Evaluation standards

A: A transparent appearance was maintained.
B: An appearance became cloudy.
C: A precipitate was observed.

**[Table 2]**

| | | | | | | | | | |
|---|---|---|---|---|---|---|---|---|---|
| MgCl₂ concentration (mM) | 0.15 | 0.2 | 0.3 | 0.4 | 0.6 | 1.2 | 3 | 6 | 12 |
| Mole ratio of MgCl₂ to TPNa | 0.05 | 0.067 | 0.1 | 0.133 | 0.2 | 0.4 | 1 | 2 | 4 |
| Determination | A | | | B | | | | C | |

### Example 8

An experiment was performed in the same manner as in Example 7 except that the final concentration of TPNa was set to 0.1 mM and the final concentration of MgCl₂ was set to 0.15 mM, 0.2 mM, 0.3 mM, 0.4 mM, 0.6 mM, or 1.2 mM. The results were summarized in Table 3.

**[Table 3]**

| | | | | | | |
|---|---|---|---|---|---|---|
| MgCl₂ concentration (mM) | 0.15 | 0.2 | 0.3 | 0.4 | 0.6 | 1.2 |
| Mole ratio of MgCl₂ to TPNa | 1.5 | 2 | 3 | 4 | 6 | 12 |
| Determination | A | | | | | |

From the results of Examples 7 and 8, it was confirmed that the mole ratio of the di- or higher valent metal salt to TPNa is preferably in a range of 0.05 to 12. It was confirmed that in a case where the concentration of TPNa is high, the mole ratio of the di- or higher valent metal salt to TPNa is preferably set to 1 or less.

### Example 9

First, a mixed liquid containing 3 mM TPNa and 0.3 mM MgCl₂ was obtained in the same manner as in Example 1. The particle diameter average of a self-assembled structure at this time was 58.07 nm. This mixed liquid was frozen at -35°C in a freezer one night and put into a freeze dryer (EYELA FDU 2100) for two days, thereby obtaining a white powder. The water content of the powder was measured by a Karl Fischer method using an automatic water content measuring instrument (HIRANUMA, AQV-300) and found out to be 0.6 mass%. The total amount of this powder was dissolved again in the same amount of water as before the drying, and the average particle diameter was then measured. It was confirmed that a redissolved composition contained a self-assembled structure having an average particle diameter of 89.78 nm. Ethanol was added to the obtained composition by the same method as in Example 1, the average particle diameter in the self-assembled structure was measured by the same method as described above, and the average particle diameter was found out to be 85.32 nm. From this results, it was confirmed that in the redissolved composition, the self-assembled structure of TPNa is stably maintained.

### Example 10

An experiment was performed by the same procedure as in Example 9 except that 0.044 g of CaCl₂·2H₂O was used instead of MgCl₂·6H₂O. The particle diameter average in the self-assembled structure before freeze drying was 123.63 nm. A white powder having a water content of 0.5 mass% was obtained by freeze drying. A composition containing a self-assembled structure having a particle diameter average of 149.70 nm was obtained by redissolution. Ethanol was added to the obtained composition by the same method as in Example 2, and the average particle diameter was found out to be 140.75 nm. From this result, it was confirmed that in the redissolved composition, the self-assembled structure of TPNa is stably maintained.

Hitherto, preferable examples of the present invention have been described, but the present invention is not limited to these examples. Addition, omission, substitution, and other modifications of the configuration are possible within the scope of the gist of the present invention. The present invention is not limited by the description described above and is only limited by the scope of the attached claims.

## Claims

1. A composition comprising:
a self-assembled structure of a tocopheryl phosphate;
a di- or higher valent metal salt; and
water.

2. The composition according to Claim 1,
wherein the di- or higher valent metal salt is at least one or more selected from the group consisting of a calcium salt, a magnesium salt, and a zinc salt.

3. The composition according to Claim 1 or 2,
wherein a concentration of the tocopheryl phosphate is 0.001 mM to 100 mM, and
a mole ratio of the di- or higher valent metal salt to the tocopheryl phosphate is 0.05 to 12.

4. A composition comprising:
a self-assembled structure of a tocopheryl phosphate; and
a di- or higher valent metal salt,
wherein a mole ratio of the di- or higher valent metal salt to the tocopheryl phosphate is 0.05 to 12, and
a moisture content is 1.0 mass% or less.

5. A freeze-dried product of the composition according to Claim 1 or 2.
